(19) Europäisches Patentamt
European Patent Office
Office européen des brevets

(11) **EP 1 833 542 B1**

(12) **EUROPÄISCHE PATENTSCHRIFT**

(45) Veröffentlichungstag und Bekanntmachung des Hinweises auf die Patenterteilung:
**28.05.2008 Patentblatt 2008/22**

(21) Anmeldenummer: 05822551.7

(22) Anmeldetag: **16.12.2005**

(51) Int Cl.:
*A61M 15/06* (2006.01)     *A24F 47/00* (2006.01)
*H05B 3/42* (2006.01)

(86) Internationale Anmeldenummer:
**PCT/EP2005/013599**

(87) Internationale Veröffentlichungsnummer:
**WO 2006/069650 (06.07.2006 Gazette 2006/27)**

(54) **MOBILES INHALATIONSGERÄT ZUM INHALIEREN VON WIRKSTOFFEN**

PORTABLE INHALATION DEVICE FOR INHALING ACTIVE SUBSTANCES

INHALATEUR PORTABLE POUR L'INHALATION DE SUBSTANCES ACTIVES

(84) Benannte Vertragsstaaten:
**CH DE ES FR IT LI**

(30) Priorität: **22.12.2004   DE 102004061883**

(43) Veröffentlichungstag der Anmeldung:
**19.09.2007   Patentblatt 2007/38**

(73) Patentinhaber: **Vishay Electronic GmbH
95100 Selb (DE)**

(72) Erfinder:
• **HERBRICH, Ernst
95100 Selb (DE)**

• **HAMPL, Otto
96114 Hirschaid (DE)**
• **PIEPER, Norbert
95100 Selb (DE)**

(74) Vertreter: **Manitz, Gerhart
Manitz, Finsterwald & Partner GbR
Martin-Greif-Strasse 1
80336 München (DE)**

(56) Entgegenhaltungen:
EP-A- 0 430 559          WO-A-03/012565
WO-A-20/04098324       DE-U1- 29 604 359
US-A- 5 878 752          US-A1- 2003 132 219

**Beschreibung**

Die Erfindung betrifft ein mobiles Inhalationsgerät zum Inhalieren von Wirkstoffen, mit einer Heizeinrichtung, die einen thermischen Speicher zum Erwärmen von Luft aufweist, die entlang des thermischen Speichers strömt, wobei der thermische Speicher mittels eines Heizdrahts der Heizeinrichtung erwärmbar ist.

Ein derartiges Inhalationsgerät gestattet das Inhalieren von Wirkstoffen, insbesondere von medizinischen Drogen und/oder Aromen. Der Benutzer eines solchen Inhalationsgeräts saugt Luft an, die zunächst entlang des thermischen Speichers strömt und hierdurch erwärmt wird. Danach strömt die erwärmte Luft entlang eines Wirkstoffdepots, wobei in dem Wirkstoffdepot befindliche Wirkstoffe durch die erwärmte Luft freigesetzt, von der erwärmten Luft aufgenommen und schließlich vom Benutzer eingeatmet werden.

Das Dokument WO 2004/098324 A2 wird als nächstliegender Stand der Technik angesehen. Es offenbart ein mobiles Inhalationsgerätes mit den Merkmalen des Oberbegriffes des Anspruchs 1. WO 2004/098324 A2 offenbart insbesondere eine rauchfreie Zigarette. Diese besitzt ein austauschbares Teil (Nikotindepot), ein wieder verwendbares Teil mit Hülse und Heizeinrichtung, sowie eine Vorrats- und Aufladestation mit Akkumulator und Thermostat. Die Heizeinrichtung des wiederverwendbaren Teils besitzt eine Heizspirale und ein wärmespeicherndes Medium, wobei zwischen dem wärmespeichernden Medium und der Hülse Luftströmungskanäle vorgesehen sind. Das wiederverwendbare Teil kann zum Zwecke des Aufheizens in der Vorrats- und Aufladestation aufge-nommen werden, wobei die Heizspirale des wiederverwendbaren Teils solange erwärmt wird, bis der Thermostat den Aufheizungsvorgang beendet.

Durch Beenden des Aufheizungsvorganges aufgrund eines entsprechenden Signals eines Thermostats sind nicht immer ein optimaler Wärmeübergang auf den thermischen Speicher und ein genaues Erreichen der Solltemperatur gewährleistet.

Aus der EP-A-0 430 559 ist ein mobiles Inhalationsgerät bekannt, bei dem ein Wirkstoffdepot auf einer relativ konstanten Betriebstemperatur gehalten wird.

Die US-A-5 878 752 beschreibt ein mobiles Inhalationsgerät mit einer Heizspirale, die zu Reinigungszwecken verwendet wird.

Es ist eine Aufgabe der Erfindung, die Erwärmung des thermischen Speichers einer Heizeinrichtung in einem Inhalationsgerät der erläuterten Art zu verbessern.

Diese Aufgabe wird durch ein Inhalationsgerät mit den Merkmalen des Anspruchs 1 gelöst.

Der Heizdraht der Heizeinrichtung besitzt einen hohen Temperaturkoeffizienten. Dadurch ist, wie nachfolgend noch erläutert wird, eine einfache und zuverlässige Bestimmung der momentanen Temperatur des thermischen Speichers möglich, ohne dass zusätzliche Temperatursonden in dem thermischen Speicher erforderlich sind. Vielmehr dient der Heizdraht selbst als Temperatursensor ("thermisches Feedback").

Als Temperaturkoeffizient ($\alpha$) wird das Verhältnis der relativen Änderung des elektrischen Widerstandes zur Temperaturänderung gemäß der nachfolgenden Formel bezeichnet:

$$\alpha = \frac{\Delta R}{R \cdot \Delta T} \, ,$$

wobei $\Delta R$ die Änderung des Widerstands (oder des spezifischen Widerstands), R den Widerstand (oder spezifischen Widerstand) und $\Delta T$ die Temperaturänderung bezeichnen. Der Temperaturkoeffizient charakterisiert also die Abhängigkeit des Widerstands R von der Temperatur T.

Demgegenüber besitzen die Heizdrähte bei bekannten Heizeinrichtungen einen vergleichsweise geringen Temperaturkoeffizienten, um eine geringe Abhängigkeit des elektrischen Widerstandes des Heizdrahts von der Temperatur und somit eine im Wesentlichen temperaturunabhängige Heizleistung zu erhalten. Beispielsweise besitzt die Legierung Konstantan einen Temperaturkoeffizienten von ca. 0,00004 K$^{-1}$.

Um den Heizdraht aufzuheizen, wird diesem elektrische Energie, also ein elektrischer Strom einer bestimmten Stärke, zugeführt. Um zugleich die momentane Temperatur des thermischen Speichers bzw. des Heizdrahts zu ermitteln, wird der momentane Wert des elektrischen Widerstands des Heizdrahts gemessen. Das Messergebnis kann dazu verwendet werden, aufgrund des ermittelten Widerstandes die weitere Zufuhr der elektrischen Energie zu regeln, also beispielsweise die Stromstärke zu verringern oder zu erhöhen oder die Energiezufuhr vollständig abzuschalten. Aufgrund des hohen Temperaturkoeffizienten des Heizdrahts (große Steigung der R/T-Kurve) kann die momentane Temperatur des Heizdrahts aus dem gemessenen Widerstand mit einer hohen Genauigkeit bestimmt werden, ohne dass zusätzliche Temperatursensoren erforderlich sind.

Die genaue Einhaltung einer vorbestimmten Temperatur des Heizdrahts und somit des thermischen Speichers ist bei der Inhalation von medizinischen Wirkstoffen besonders wichtig, damit die vorgesehene Dosierung einerseits sicher erreicht und andererseits wiederum nicht übermäßig überschritten wird. Besonders wichtig ist eine genaue Temperaturbestimmung und eine entsprechend genaue Steuerung oder Regelung der Heizleistung auch für mobile Anwendungen des Inhalationsgeräts, da die Kapazität der verwendeten elektrischen Energiequelle (Akkumulator) begrenzt ist und stets optimal ausgenutzt werden soll.

Ein besonders vorteilhafter Wert des Temperaturkoeffizienten liegt beispielsweise bei 0,003 K$^{-1}$, wobei natürlich eine umso bessere Messauflösung erzielt wird, je höher der Temperaturkoeffizient ist.

Das mobile Inhalationsgerät kann ferner eine elektrische Energiequelle, die an dem Heizdraht anschließbar ist, und eine Auswerte- und Steuerschaltung aufweisen, durch die der elektrische Widerstand des Heizdrahts ermittelbar ist und durch die anhand des ermittelten Widerstands die elektrische Energie einstellbar ist, die dem Heizdraht zugeführt wird.

Weitere Ausführungsformen der Erfindung sind in den Unteransprüchen beschrieben.

Ein erfindungsgemäßes Inhalationsgerät wird nachfolgend lediglich beispielhaft unter Bezugnahme auf die Zeichnungen erläutert, wobei die Fig. 1 bis 6 eine Heizeinrichtung für ein solches Inhalationsgerät zeigen.

Fig. 1     zeigt eine Perspektivansicht einer Heizeinrichtung.

Fig. 2     zeigt eine Perspektivansicht von Teilen der Heizeinrichtung.

Fig. 3     zeigt eine aufgeschnittene Perspektivansicht der Heizeinrichtung.

Fig. 4     ist eine Detailansicht des Bereichs IV gemäß Fig. 3.

Fig. 5     zeigt die Heizeinrichtung im Längsschnitt.

Fig. 6     ist eine Detailansicht des Bereichs VI gemäß Fig. 5.

Fig. 7     zeigt ein mobiles Inhalationsgerät mit der Heizeinrichtung.

Fig. 8     zeigt die Abhängigkeit des Widerstands der Heizspirale von der Temperatur.

Fig. 9     zeigt ein Blockschaltbild der Verschaltung einer Heizspirale und einer elektrischen Energiequelle mit einer Auswerte- und Steuerschaltung.

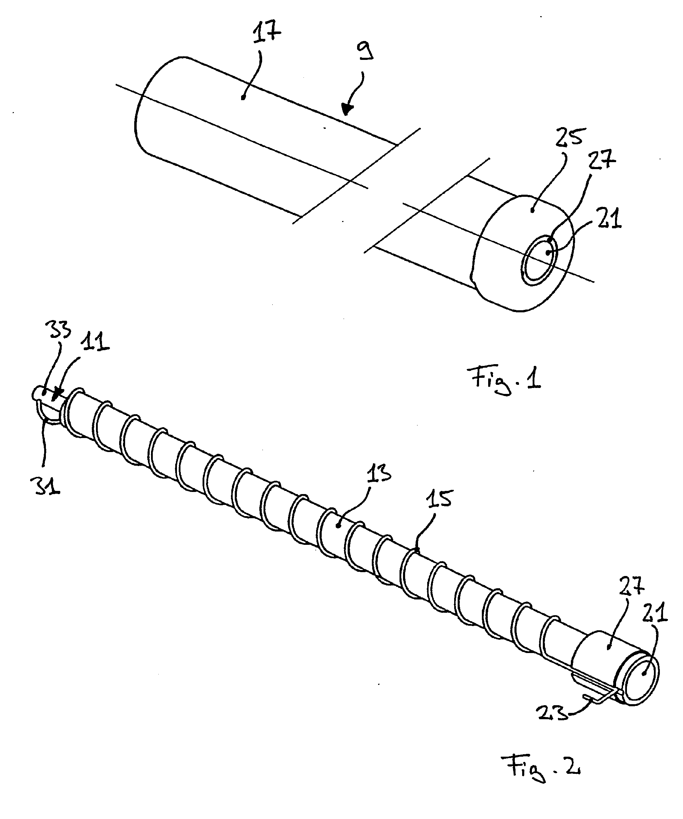

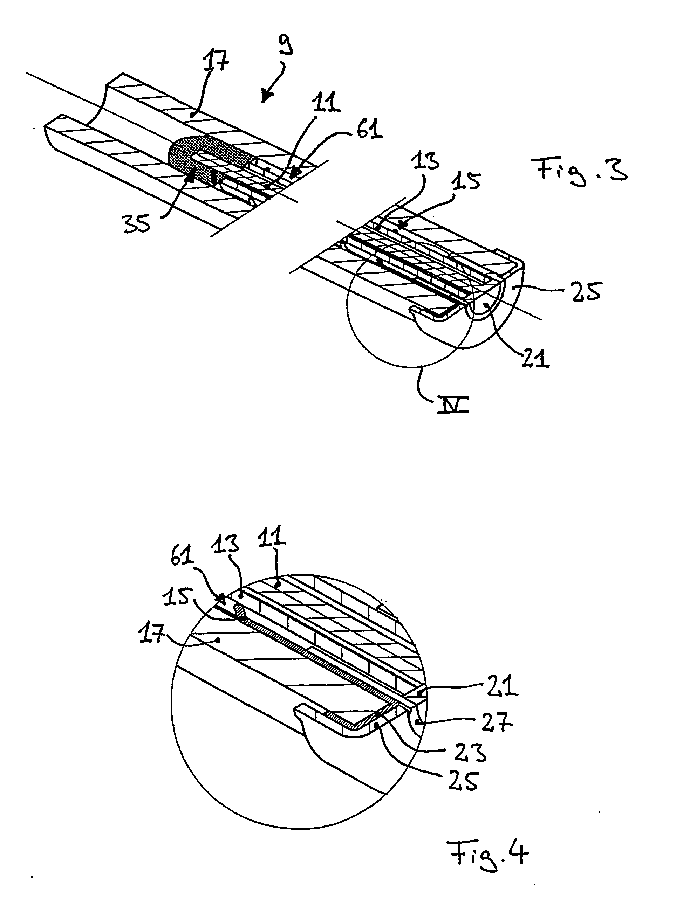

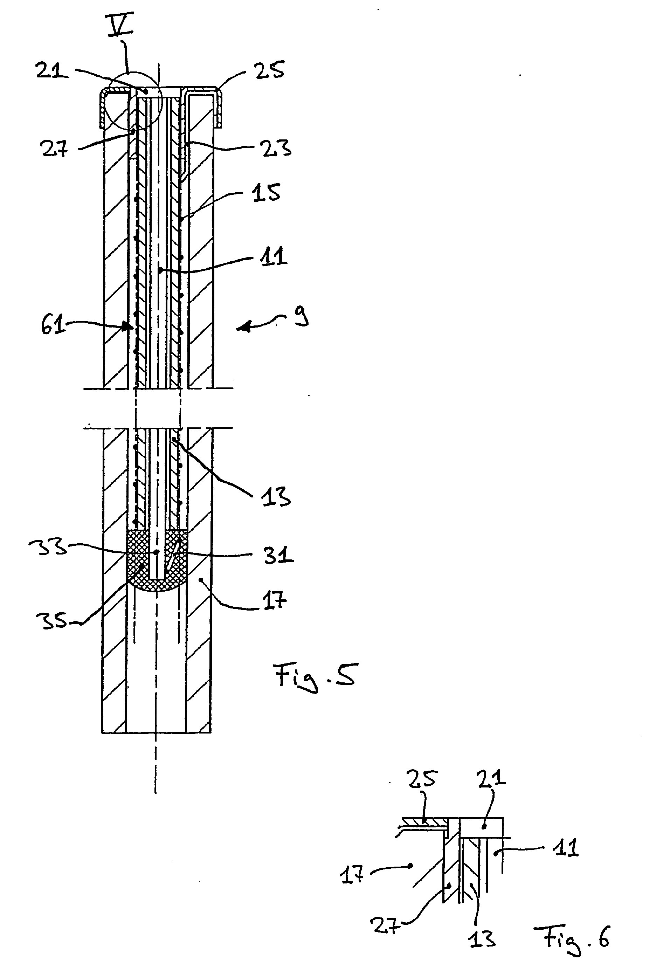

Die in den Fig. 1 bis 6 gezeigte Heizeinrichtung 9 weist eine Längsform mit nahezu rotationssymmetrischem Aufbau auf. Die Heizeinrichtung 9 besitzt- radial von innen nach außen - einen Mittelkontaktstift 11, ein keramisches Innenrohr 13, eine Heizspirale 15 und ein keramisches Außenrohr 17 (vgl. insbesondere Fig. 3 und 5). Der Mittelkontaktstift 11, das Innenrohr 13, die Heizspirale 15 und das Außenrohr 17 sind koaxial zueinander angeordnet. Der Mittelkontaktstift 11 besitzt eine hohe elektrische Leitfähigkeit und besteht beispielsweise aus Kupfer-Nickel oder anderen metallischen Legierungen.

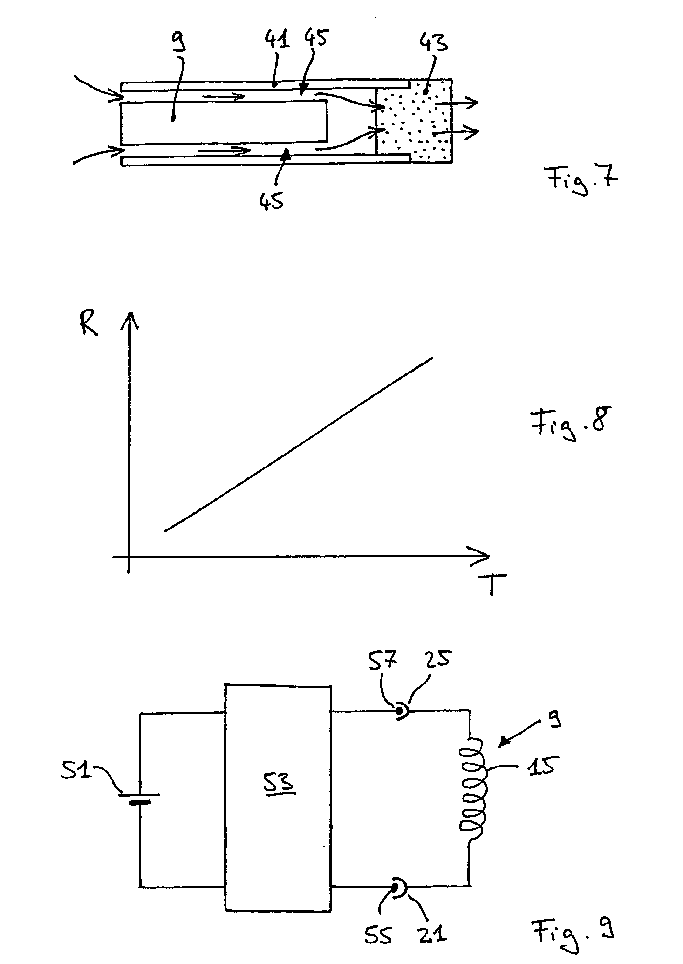

Die Heizspirale 15 besitzt demgegenüber einen hohen elektrischen Widerstand. Sie dient zum Erwärmen des Innenrohrs 13 und des Außenrohrs 17 und besitzt einen hohen Temperaturkoeffizienten, wie nachfolgend noch erläutert wird (Fig. 8). Beispielsweise kann die Heizspirale 15 aus der Nickel-Eisen-Legierung "Resistherm" (eingetragene Marke) mit den Bestandteilen Aluminium (Massenanteil 0,6%), Chrom (0,3%), Eisen (30%), Mangan (0,5%) und Nickel (Rest) bestehen, mit einem Temperaturkoeffizienten von ca. 0,003 K$^{-1}$. Das Innenrohr 13 und das Außenrohr 17 bilden gemeinsam einen thermischen Speicher für die von der Heizspirale abgegebene Wärme, und sie bestehen aus einem keramischen Material, beispielsweise Titanat oder Steatit.

An einem vorderseitigen Ende des Mittelkontaktstifts 11 (in Fig. 3 rechts, in Fig. 5 oben) ist ein kreisscheibenförmiger Kontaktsockel 21 angeformt. Die Vorderseite des keramischen Innenrohrs 13 liegt an der Rückseite dieses Kontaktsockels 21 an. Ein vorderseitiges Anschlussende 23 der Heizspirale 15 ist U-förmig umgebogen, wobei die U-Form die Vorderseite des keramischen Außenrohrs 17 umgreift (vgl. Fig. 2 und 3). Auf das vorderseitige Ende des Außenrohrs 17 ist durch Presspassung ein Ringkontakt 25 aus Kupfer oder einer metallischen Legierung mit leitfähigem Überzug derart aufgesetzt, dass das vorderseitige Anschlussende 23 der Heizspirale 15 an der Innenseite des Ringkontakts 25 anliegt und zwischen diesen beiden Teilen somit eine elektrische Verbindung hergestellt ist.

Zwischen dem vorderseitigen Ende des Mittelkontaktstifts 11 mit Kontaktsockel 21 und dem vorderseitigen Ende des keramischen Innenrohrs 13 einerseits, und dem vorderseitigen Ende des keramischen Außenrohrs 17 mit aufgesetztem Ringkontakt 25 andererseits ist eine Isolatorhülse 27 angeordnet, die den Kontaktsockel 21 und den Ringkontakt 25 voneinander elektrisch isoliert. Bei der gezeigten Ausführungsform schließen der Kontaktsockel 21, die Isolatorhülse 27 und der Ringkontakt 25 an der Vorderseite der Heizeinrichtung 9 bündig miteinander ab (vgl. Fig. 3 und 5). Der Kontaktsockel 21, die Isolatorhülse 27 und der Ringkontakt 25 sind koaxial zueinander angeordnet.

Ein rückseitiges Anschlussende 31 der Heizspirale 15 ist mit einem rückseitigen Ende 33 des Mittelkontaktstifts 11 elektrisch verbunden. Dieses rückseitige Ende 33 des Mittelkontaktstifts 11 ragt aus dem keramischen Innenrohr 13 heraus, welches kürzer als der Mittelkontaktstift 11 und kürzer als das keramische Außenrohr 17 ist. Das rückseitige Ende 33 des Mittelkontaktstifts 11 und das rückseitige Anschlussende 31 der Heizspirale 15 sind mittels einer Befestigungsmasse 35 an der Innenseite des keramischen Außenrohrs 17 fixiert. Bei der Befestigungsmasse 35 kann es sich beispielsweise um einen Zement handeln, der von der Rückseite aus in den Innenraum des Außenrohrs 17 eingegossen ist. Das keramische Innenrohr 13 ist zwischen der Befestigungsmasse 35 einerseits und dem Kontaktsockel 21 des Mittelkontaktstifts 11 andererseits gefangen.

Die in den Fig. 1 bis 6 gezeigte Heizeinrichtung 9 ist zum Einsatz in einem mobilen Inhalationsgerät zum Inhalieren von Wirkstoffen vorgesehen, wobei an der Heizeinrichtung 9 vorbeiströmende Luft erwärmt wird und Wirkstoffe aus einem Wirkstoffdepot freigesetzt werden, die dann inhaliert werden können.

Fig. 7 zeigt in einer schematischen Querschnittsansicht Teile eines solchen mobilen Inhalationsgeräts. Dieses Inhalationsgerät besitzt eine Verbindungshülse 41, die die Heizeinrichtung 9 umfänglich umgibt und an einem Ende mit einem luftdurchlässigen Wirkstoffdepot 43 verbunden ist. Zwischen der Verbindungshülse 41 und der Heizeinrichtung 9 sind ein oder mehrere Strömungskanäle 45 ausgebildet. Der Benutzer des Inhalationsgeräts setzt mit dem Mund an dem Wirkstoffdepot 43 an und saugt Luft an. Die angesaugte Luft strömt durch die Strömungskanäle 45 entlang der Heizeinrichtung 9 (vgl. Pfeile in Fig. 7). Die Luft strömt somit entlang des keramischen Außenrohrs 17 (Fig. 1, 3, 5), das zuvor mittels der Heizspirale 15 erwärmt worden ist. Die somit erwärmte Luft strömt danach durch das Wirkstoffdepot 43, erwärmt dieses und setzt dadurch in dem Wirkstoffdepot 43 gelagerte Wirkstoffe frei, die von der Luft aufgenommen und mitgeführt werden. Der Benutzer atmet diese Wirkstoffe nun also ein.

Für viele Anwendungen eines derartigen Inhalationsgeräts ist es wichtig, dass die Wirkstoffe in einer vorbestimmten Menge und/oder möglichst gleichmäßig während einer vorbestimmten Dauer freigesetzt werden. Hierfür ist es wiederum wichtig, dass die Temperatur der Heizspirale 15, mit der der thermische Speicher (Innenrohr 13 und Außenrohr 17) erwärmt wird, möglichst genau eingestellt werden kann. Daher ist es wünschenswert, dass die tatsächliche momentane Temperatur der Heizspirale 15 ermittelt werden kann.

Zu diesem Zweck ist vorgesehen, dass die Heizspirale 15 aus einem Material besteht, das - wie bereits erwähnt - einen hohen Temperaturkoeffizienten besitzt, d.h. der elektrische Widerstand der Heizspirale 15 hängt stark von deren Temperatur ab. Fig. 8 zeigt beispielhaft eine solche Abhängigkeit des Widerstands R der Heizspirale 15 von ihrer Temperatur T. Charakteristisch für das zu verwendende Material der Heizspirale 15 ist die große Steigung des in Fig. 8 gezeigten Graphen. Aufgrund der starken Temperaturabhängigkeit des Widerstands kann aus einem ermittelten Widerstandswert mit hoher Genauigkeit der entsprechende Temperaturwert bestimmt werden.

Fig. 9 zeigt weitere Teile des bereits beschriebenen mobilen Inhalationsgeräts. Dieses besitzt einen Akkumulator 51 oder eine Batterie als elektrische Energiequelle sowie eine Auswerte- und Steuerschaltung 53, die mit den beiden Polen des Akkumulators 51 verbunden ist. Die Auswerte- und Steuerschaltung 53 besitzt ausgangsseitig zwei Verbindungskontakte 55, 57. Die Auswerte- und Steuerschaltung 53 dient als Treiber für die Energiezufuhr von dem Akkumulator 51 zu der Heizspirale 15. Die Heizeinrichtung 9, von der in Fig. 9 lediglich die Heizspirale 15 gezeigt ist, wird mittels des Kontaktsockels 21 und des Ringkontakts 25 zeitweise mit den Verbindungskontakten 55, 57 verbunden, wobei ein elektrischer Strom durch die Heizspirale 15 geführt wird, um diese zu erwärmen.

Dabei steuert die Auswerte- und Steuerschaltung 53 den Strom, der durch die Heizspirale 15 geführt wird, in Abhängigkeit von der Temperatur der Heizspirale 15. Hierfür misst die Auswerte- und Steuerschaltung 53 den elektrischen Widerstand R der Heizspirale 15. Aufgrund der starken Temperaturabhängigkeit des Widerstands R (vgl. Fig. 8) ergibt sich die Temperatur T hieraus mit großer Genauigkeit.

Nachfolgend werden Vorteile der Heizeinrichtung gemäß Fig. 1 bis 6 und des Inhalationsgeräts gemäß Fig. 7 und 9 erläutert:

a) Der thermische Speicher besitzt sowohl ein Außenteil (Außenrohr 17), das außerhalb der Heizspirale 15 angeordnet ist, als auch ein Innenteil (Innenrohr 13), das innerhalb der Heizspirale 15 angeordnet ist. Hierdurch wird nach dem Erwärmen des thermischen Speichers mittels der Heizspirale 15 eine gleichmäßige Wärmeabgabe über einen langen Zeitraum ermöglicht, und die Wärmeabgabe erweist sich als weniger empfindlich gegenüber unterschiedlichen Umgebungstemperaturen des thermischen Speichers.

b) Gemäß einer Weiterbildung der vorgenannten Ausführungsform ist es bevorzugt, wenn die Heizspirale 15 an der Außenseite des Innenteils des thermischen Speichers (Innenrohr 13) anliegt und wenn zwischen der Heizspirale 15 und der Innenseite des Außenteils des thermischen Speichers (Außenrohr 17) zumindest entlang eines Längsabschnitts der Heizeinrichtung 9 ein Luftspalt 61 vorgesehen ist (vgl. Fig. 5). Ein derartiger Luftspalt 61 trägt ebenfalls zur Verbesserung der Gleichmäßigkeit der Wärmeabgabe bei. Aufgrund des Luftspalts 61 ist der Wärmeübergang von der Heizspirale 15 zum Außenrohr 17 nämlich schlechter als zum Innenrohr 13. Dies hat nach der Unterbrechung der Energieversorgung der Heizspirale 15 zur Folge, dass die Wärmeabgabe vom Innenrohr 13 zum Außenrohr 17 verzögert wird und das Innenrohr 13 für das Außenrohr 17 als thermischer Speicher auf einem höheren Temperaturniveau wirkt. Während das Außenrohr 17 Wärme nach Außen abgibt, kann das Innenrohr 13 das Außenrohr 17 somit länger "nachheizen".

c) Da das rückseitige Anschlussende 31 der Heizspirale 15, das rückseitige Ende 33 des Mittelkontaktstifts 11 und somit auch das rückseitige Ende des Innenrohrs 13 mittels der Befestigungsmasse 35 gemeinsam lagefixiert sind, kann die Heizeinrichtung 9 - bei im Wesentlichen unverändertem Herstellungsverfahren - auch in einer Ausführung gefertigt werden, bei der der Kontaktsockel 21 des Mittelkontaktstifts 11 bezüglich der Vorderseite des Außenrohrs 17 und somit bezüglich des Ringkontakts 25 zurückversetzt ist. Mit anderen Worten kann alternativ zu der in den Fig. 3 und 5 gezeigten Ausführungsform ein Stifteingriffskontakt verwirklicht werden, durch den ein elektrischer Kurzschluss an der verwendeten Energiequelle sicherer vermieden werden kann. Ein derartiger Stifteingriffskontakt ist nicht in allen Fällen erforderlich oder erwünscht. Ein besonderer Vorteil der erläuterten Ausgestaltung der Heizeinrichtung 9 besteht jedoch darin, dass beide Varianten (bündiger oder zurückversetzter Kontaktsockel 21) mit im Wesentlichen demselben Herstellungsverfahren realisiert werden können.

d) Der Kontaktsockel 21 und der Ringkontakt 25 besitzen einen rotationssymmetrischen Aufbau (vgl. Fig. 1). Hierdurch ist eine Verbindung mit der verwendeten elektrischen Energiequelle in einer beliebigen Winkelstellung der Heizeinrichtung 9 möglich, was den Bedienungskomfort für den Benutzer verbessert.

e) Die Auswerte- und Steuerschaltung 53 (Fig. 9) kann für eine Regelung der Energiezufuhr zu der Heizspirale 15 ausgebildet sein, wobei die der Heizspirale 15 zugeführte elektrische Energie gemäß einem vorbestimmten zeitlichen Verlauf der Temperatur T (bzw. des Widerstands R) der Heizspirale 15 eingestellt wird. Mit anderen Worten ist nicht lediglich vorgesehen, dass das Erwärmen der Heizspirale 15 bei Erreichen einer vorbestimmten Temperatur beendet wird, sondern es ist auch vorgesehen, dass der Erwärmungsvorgang einem vorbestimmten zeitlichen Verlauf folgt. Hierdurch kann die Ausnutzung der Kapazität des Akkumulators 51 optimiert werden.

Bezugszeichenliste

9  Heizeinrichtung
11  Mittelkontaktstift
13  Innenrohr
15  Heizspirale
17  Außenrohr
21  Kontaktsockel
23  vorderseitiges Anschlussende der Heizspirale
25  Ringkontakt
27  Isolatorhülse
31  rückseitiges Anschlussende des Heizspirale
33  rückseitiges Ende des Mittelkontaktstifts
35  Befestigungsmasse
41  Verbindungshülse
43  Wirkstoffdepot
45  Strömungskanal
51  Akkumulator

53  Auswerte- und Steuerschaltung
55  Verbindungskontakt
57  Verbindungskontakt
61  Luftspalt

R  Widerstand
T  Temperatur

## Patentansprüche

1. Mobiles Inhalationsgerät zum Inhalieren von Wirkstoffen, mit einer Heizeinrichtung (9) und einer Verbindungshülse (41), die die Heizeinrichtung (9) umfänglich umgibt und an einem Ende mit einem luftdurchlässigen Wirkstoffdepot (43) verbunden oder verbindbar ist, wobei zwischen der Verbindungshülse (41) und der Heizeinrichtung (9) wenigstens ein Strömungskanal (45) ausgebildet ist, wobei die Heizeinrichtung (9) einen als eine Heizspirale (15) ausgebildeten Heizdraht aufweist, der zwei Anschlussenden (23, 31) zum Zuführen von elektrischer Energie aufweist, und wobei die Heizeinrichtung (9) einen thermischen Speicher (13, 17) zum Erwärmen von Luft aufweist, die entlang des thermischen Speichers strömt, wobei der thermische Speicher mittels des Heizdrahts erwärmbar ist, wobei die von einem Benutzer durch den wenigstens einen Strömungskanal (45) angesaugte Luft entlang der gesamten Länge des Außenteils (17) des thermischen Speichers strömt,
**dadurch gekennzeichnet, dass** der Heizdraht (15) einen Temperaturkoeffizienten von wenigstens 0,001 K$^{-1}$ besitzt und wobei ein Außenteil (17) des thermischen Speichers außerhalb der Heizspirale und ein Innenteil (13) des thermischen Speichers innerhalb der Heizspirale angeordnet sind.

2. Inhalationsgerät nach Anspruch 1,
**dadurch gekennzeichnet,**
**dass** der Heizdraht (15) einen Temperaturkoeffizienten von ca. 0,003 K$^{-1}$ besitzt.

3. Inhalationsgerät nach einem der vorhergehenden Ansprüche,
**dadurch gekennzeichnet,**
**dass** der Heizdraht (15) aus einer Nickel-Eisen-Legierung besteht.

4. Inhalationsgerät nach einem der vorhergehenden Ansprüche,
**dadurch gekennzeichnet,**
**dass** wenigstens das Außenteil (17) des thermischen Speichers hohlzylindrisch ausgebildet ist,
**dass** die Heizspirale (15) sich entlang der Längsachse des hohlzylindrischen thermischen Speichers (17) erstreckt, wobei ein erstes Anschlussende (23) der Heizspirale (15) an oder benachbart zu einem ersten Ende des hohlzylindrischen thermischen Speichers (17) und ein zweites Anschlussende (31) der Heizspirale (15) an oder benachbart zu einem zweiten Ende des hohlzylindrischen thermischen Speichers (17) angeordnet sind, und

**dass** die Heizeinrichtung ferner einen Mittelkontaktstift (11) aufweist, der innerhalb des hohlzylindrischen thermischen Speichers (17) angeordnet ist, mit einem ersten Ende (21) an oder benachbart zu dem ersten Ende des hohlzylindrischen thermischen Speichers (17) angeordnet ist und mit einem zweiten Ende (33) an oder benachbart zu dem zweiten Ende des hohlzylindrischen thermischen Speichers (17) angeordnet ist, wobei das zweite Ende (33) des Mittelkontaktstifts (11) mit dem zweiten Anschlussende (31) der Heizspirale (15) elektrisch verbunden ist und an der Innenseite des hohlzylindrischen thermischen Speichers (17) befestigt ist.

5.  Inhalationsgerät nach Anspruch 4,
    **dadurch gekennzeichnet,**
    **dass** ein Anschlussende (23) des Heizdrahts (15) einen Ringkontakt aufweist, der ein Ende (21) des Mittelkontaktstifts (11) koaxial zu dem Mittelkontaktstift umgibt.

6.  Inhalationsgerät nach einem der vorhergehenden Ansprüche,
    **dadurch gekennzeichnet,**
    **dass** das Außenteil (17) und das Innenteil (13) des thermischen Speichers jeweils hohlzylindrisch ausgebildet und koaxial zueinander angeordnet sind.

7.  Inhalationsgerät nach einem der vorhergehenden Ansprüche,
    **dadurch gekennzeichnet,**
    **dass** die Heizspirale (15) an der Außenseite des Innenteils (13) des thermischen Speichers anliegt, und
    **dass** zwischen der Heizspirale (15) und der Innenseite des Außenteils (17) des thermischen Speichers zumindest abschnittsweise ein Luftspalt (61) vorgesehen ist.

8.  Inhalationsgerät nach einem der vorhergehenden Ansprüche,
    **dadurch gekennzeichnet,**
    **dass** der oder die thermischen Speicher (13, 17) aus einer Keramik besteht.

9.  Inhalationsgerät nach einem der vorhergehenden Ansprüche,
    **gekennzeichnet**
    **durch** eine elektrische Energiequelle (51), die an den Heizdraht (15) anschließbar ist, und durch eine Auswerte- und Steuerschaltung (53), durch die der elektrische Widerstand (R) des Heizdrahts (15) ermittelbar ist und durch die anhand des ermittelten Widerstands die elektrische Energie einstellbar ist, die dem Heizdraht zugeführt wird.

10. Inhalationsgerät nach einem der vorhergehenden Ansprüche,
    **dadurch gekennzeichnet,**
    **dass** das Inhalationsgerät das Wirkstoffdepot (43) aufweist, wobei das Wirkstoffdepot (43) dergestalt benachbart zu dem thermischen Speicher (13, 17) der Heizeinrichtung (9) angeordnet ist, dass die entlang des thermischen Speichers strömende und hierdurch erwärmte Luft entlang des Wirkstoffdepots geführt wird, um in dem Wirkstoffdepot befindliche Wirkstoffe aufzunehmen.

## Claims

1.  A mobile inhaler for the inhalation of active agents having a heating device (9) and a connection sleeve (41) which surrounds the periphery of the heating device (9) and is connected or connectable at one end to an air permeable active agent depot, with a flow passage (45) being formed between the connection sleeve (41) and the heating device (9), with the heating device (9) having a hearing wire formed as a heating spiral (15) which has two connection ends (23, 31) for the supply of electrical energy and wherein the heating device (9) has a thermal reservoir (13, 17) for the heating of air which flows along the thermal reservoir, with the thermal reservoir being capable of being heated by means of the heating wire and with the air sucked in by a user through the at least one flow passage (45) flowing along the entire length of the outer part (17) of the thermal reservoir,
    **characterized in that**
    the heating wire (15) has a temperature coefficient of at least 0.001 $K^{-1}$ and wherein an outer part (17) of the thermal reservoir is arranged outside of the heating spiral and an inner part (13) of the thermal reservoir is arranged inside the heating spiral.

2.  An inhaler in accordance with claim 1, **characterized in that** the heating wire (15) has a temperature coefficient of approx. 0.003 $K^{-1}$.

3.  An inhaler in accordance with one of the preceding claims, **characterized in that** the heating wire (15) comprises a nickel-iron alloy.

4.  An inhaler in accordance with any one of the preceding claims, **characterized in that** at least the outer part (17) of the thermal reservoir is of hollow cylindrical shape; **in that** the heating spiral (15) extends along the longitudinal axis of the hollow cylindrical thermal reservoir (17), with a first connection end (23) of the heating spiral (15) being arranged at or adjacent to a first end of the hollow cylindrical thermal reservoir (17) and a second connection end (31) of the heating spiral (15) being arranged at or adjacent to a second end of the hollow cylindrical thermal reservoir (17); and **in that** the heating device further has a center contact pin (11) which is arranged inside the hollow cylindrical thermal reservoir (17), with a first end (21) arranged at or adjacent to the first end of the hollow cylindrical thermal reservoir (17) and with a second end (33) arranged at or adjacent to the second end of the hollow cylindrical thermal reservoir (17), with the second end (33) of the center contact pin (11) being electrically connected to the second connection end (31) of the heating spiral (15) and being fastened to the inner side of the hollow cylindrical thermal reservoir (17).

**5.** An inhaler in accordance with claim 4, **characterized in that** a connection end (23) of the heating wire (15) has a ring contact which surrounds an end (21) of the center contact pin (11) coaxially to the center contact pin.

**6.** An inhaler in accordance with any one of the preceding claims, **characterized in that** the outer part (17) and the inner part (13) of the thermal reservoir are each of hollow cylindrical shape and are arranged coaxially to one another.

**7.** An inhaler in accordance with any one of the preceding claims, **characterized in that** the heating spiral (15) contacts the outer side (13) of the inner part (13) of the thermal reservoir; and **in that** an air gap (61) is provided at least sectionally between the heating spiral (15) and the inner side of the outer part (17) of the thermal reservoir.

**8.** An inhaler in accordance with any one of the preceding claims, **characterized in that** the thermal reservoir or reservoirs (13, 17) comprise a ceramic material.

**9.** An inhaler in accordance with any one of the preceding claims, **characterized by** an electrical energy source (51) which is connectable to the heating wire (15) and by an evaluation and control circuit (53) by which the electrical resistance (R) of the heating wire (15) can be determined and by which the electrical energy supplied to the heating wire can be set with reference to the determined resistance.

**10.** An inhaler in accordance with any one of the preceding claims, **characterized in that** the inhalation unit has an active agent depot (43) which is arranged adjacent to the thermal reservoir (13, 17) of the heating device (9) such that the air flowing along the thermal reservoir and hereby being heated is guided along the active agent depot to take up active agents located in the active agent depot.

**Revendications**

**1.** Inhalateur mobile pour l'inhalation de substances actives, comprenant un dispositif de chauffage (9) et une douille de liaison (41), qui entoure le dispositif de chauffage (9) sur le pourtour et est reliée ou peut être reliée par une extrémité à un dépôt de substance active (43) perméable à l'air, au moins un canal d'écoulement (45) étant réalisé entre la douille de liaison (41) et le dispositif de chauffage (9), le dispositif de chauffage (9) présentant un fil de chauffage conçu comme une spirale de chauffage (15), qui présente deux extrémités de raccordement (23, 31) pour l'arrivée d'énergie électrique, et le dispositif de chauffage (9) présentant un accumulateur thermique (13, 17) pour le réchauffement de l'air qui circule le long de l'accumulateur thermique, l'accumulateur thermique pouvant être chauffé au moyen du fil de chauffage, l'air aspiré par un utilisateur par l'au moins un canal d'écoulement (45) s'écoulant le long de l'ensemble de la longueur de la partie extérieure (17) de l'accumulateur thermique, **caractérisé en ce que** le fil de chauffage (15) présente un coefficient de température d'au moins 0,001 $K^{-1}$ et une partie extérieure (17) de l'accumulateur thermique étant disposée à l'extérieur de la spirale de chauffage et une partie intérieure (13) de l'accumulateur thermique étant disposée à l'intérieur de la spirale de chauffage.

**2.** Inhalateur selon la revendication 1, **caractérisé en ce que** le fil de chauffage (15) présente un coefficient de température d'environ 0,003 $K^{-1}$.

**3.** Inhalateur selon l'une des revendications précédentes, **caractérisé en ce que** le fil de chauffage (15) est à base d'un alliage nickel-fer.

**4.** Inhalateur selon l'une des revendications précédentes, **caractérisé**
en ce qu'au moins la partie extérieure (17) de l'accumulateur thermique est conçue en forme de cylindre creux,
**en ce que** la spirale de chauffage (15) s'étend le long de l'axe longitudinal de l'accumulateur (17) thermique cylindrique creux, une première extrémité de raccordement (23) de la spirale de chauffage (15) étant disposée sur ou à proximité d'une première extrémité de l'accumulateur (17) thermique cylindrique creux et une seconde extrémité de raccordement (31) de la spirale de chauffage (15) étant disposée sur ou à proximité d'une seconde extrémité de l'accumulateur (17) thermique cylindrique creux, et
**en ce que** le dispositif de chauffage présente également une broche de contact centrale (11) qui est disposée à l'intérieur de l'accumulateur (17) thermique cylindrique creux, est disposée avec une première extrémité (21) sur, ou à proximité de, la première extrémité de l'accumulateur (17) thermique cylindrique creux et est disposée avec une seconde extrémité (33) sur, ou à proximité de, la seconde extrémité de l'accumulateur (17) thermique cylindrique creux, la seconde extrémité (33) de la broche de contact centrale (11) étant reliée électriquement à la seconde extrémité de raccordement (31) de la spirale de chauffage (15) et étant fixée sur le côté intérieur de l'accumulateur (17) thermique cylindrique creux.

5. Inhalateur selon la revendication 4, **caractérisé en ce qu'**une extrémité de raccordement (23) du fil de chauffage (15) présente un contact annulaire qui entoure une extrémité (21) de la broche de contact centrale (11) sur le même axe que la broche de contact centrale.

6. Inhalateur selon l'une des revendications précédentes, **caractérisé en ce que** la partie extérieure (17) et la partie intérieure (13) de l'accumulateur thermique sont conçues chacune en forme de cylindre creux et sont disposées sur le même axe l'une par rapport à l'autre.

7. Inhalateur selon l'une des revendications précédentes, **caractérisé**
**en ce que** la spirale de chauffage (15) s'applique sur le côté extérieure de la partie intérieure (13) de l'accumulateur thermique, et
**en ce qu'**une fente d'air (61) est prévue au moins par endroits entre la spirale de chauffage (15) et le côté intérieur de la partie extérieure (17) de l'accumulateur thermique.

8. Inhalateur selon l'une des revendications précédentes, **caractérisé en ce que** l'accumulateur ou les accumulateurs (13, 17) thermiques sont à base d'une céramique.

9. Inhalateur selon l'une des revendications précédentes, **caractérisé par** une source d'énergie (51) électrique, qui peut être raccordée au fil de chauffage (15), et par un circuit d'analyse et de commande (53), par lequel la résistance (R) électrique du fil de chauffage (15) peut être déterminée et par lequel l'énergie électrique, qui est amenée au fil de chauffage, peut être réglée à l'aide de la résistance calculée.

10. Inhalateur selon l'une des revendications précédentes, **caractérisé en ce que** l'inhalateur présente le dépôt de substance active (43), le dépôt de substance active (43) étant disposé à proximité de l'accumulateur (13, 17) thermique du dispositif de chauffage (9) de telle sorte que l'air circulant le long de l'accumulateur thermique et réchauffé par celui-ci est guidé le long du dépôt de substance active, afin d'absorber des substances actives se trouvant dans le dépôt de substance active.

17

9

25
27
21

Fig. 1

33 11

31

13

15

27

21

23

Fig. 2

Fig.3

Fig.4

Fig. 5

Fig. 6

Fig.7

Fig.8

Fig.9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004098324 A2
- EP 0430559 A
- US 5878752 A